# EUROPEAN PATENT SPECIFICATION

(11) **EP 2 944 373 B1**
(45) Date of publication and mention of the grant of the patent: **20.06.2018**
(21) Application number: 15166679.9
(22) Date of filing: 07.05.2015
(51) Int. Cl.: B01F 11/00, B01F 13/08, B01F 15/00, B01F 15/02, G01N 1/38, C12M 1/06

(54) **DEVICE FOR SUSPENDING CELLS**
EINRICHTUNG ZUM SUSPENDIEREN VON ZELLEN
DISPOSITIF DE MISE EN SUSPENSION DE CELLULES

(30) Priority: 17.05.2014 EP 14168756
(43) Date of publication of application: 18.11.2015
(73) Proprietor: Miltenyi Biotec GmbH, 51429 Bergisch Gladbach (DE)
(72) Inventor: Büscher, Martin, 51429 Bergisch Gladbach (DE); Peters, Ralf-Peter, 51429 Bergisch Gadbach (DE); Ignatius, Sven, 51465 Bergisch Gladbach (DE); Hammerschmidt, Dominik, 51789 Lindlar (DE)
(74) Representative: Kisters, Michael Marcus

(56) References cited:
- EP-A2- 0 053 869
- US-A- 4 046 515
- US-A1- 2008 078 257
- US-A1- 2012 127 821

## Description

This invention relates to a method and a device for suspending particles in a fluid, especially for suspending living cells in small volume of fluids.

### Background

Biological samples like cells need to be suspended in a liquid for culturing or analyzing purposes. Such suspensions are stable for short time until the sample starts sedimentation which makes remixing necessary. Mixing suspensions of biological samples is a long known technique for which a various devices are commercially available. Design and function of the mixing devices depend on the nature and volume of the sample to be mixed, the size and form of the mixing vessel and subsequent processing steps of the sample.

Besides mixing a suspension by a stirrer coupled to an electric motor, magnetic cell stirrers are in common use. To provide larger volumes of suspensions, EP53869, US 3854704 and US 3572651 disclose a magnetic cell stirrer wherein the suspension is mixed by a rotating magnet placed in the mixing vessel via a flexible shaft. The magnet inside of the vessel is forced into rotational movement by a magnet outside of the vessel which in turn is rotated by a conventional magnetic stirring apparatus. The flexible shaft is attached to the mixing vessel and can not be removed or inserted in the vessel.

US3780992 discloses a vibrating pipette, wherein a mechanical oscillator is attached to the pipette as mixing rod.

US 4204774 describes a magnetic cell stirrer wherein the suspension is mixed by a rotating stirring rod. The rod is rotated in a circular path in the mixing vessel by an eccentric means and a synchronous motor. The magnetic cell stirrer is firmly attached to the mixing vessel and can not be removed or inserted in the vessel.

Biological samples for testing or analyzing purposes are usually provided in small volumes ranging from less than 10 µl to 5 ml. For example, commonly used microplates provide up to 1024 wells having a volume less than one milliliter. Mixing of such small volumes by mere stirring is difficult due to capillary forces and a low surface to volume ratio of the vessel resulting in adhesion of the liquid to the vessel walls. Mixing by stirring generates a single stream of liquid moving in a circle adjacent to the vessel wall having low turbulence and accordingly a low mixing efficiency.

In this respect, US2008/0078257 discloses a mixing process, and a mixing device according to the preamble of claim 1, wherein a magnetic mixing rod is moved by a second magnet rotating around the mixing rod.

Suspensions in microplate wells are therefore usually mixed by shaking or vibrating the whole microplate i.e. all wells simultaneously. Shaking cell suspensions in a microplate might result in cell loss since cells can adhere to walls of a well above the surface of the liquid It would be beneficial to provide small volume suspensions of biological samples without stirring the fluid in circles and/or the need to shake/vibrate other vessels. It would furthermore be desirous to provide a device to generate such suspensions which can be implemented in analytical devices like a FACS machines for sample preparation upstream of the cell analysis device.

Surprisingly, it was found that particle suspensions, especially in small volume containers can be efficiently homogenized by a device wherein a magnetic mixing rod is moved in an oscillating manner by interaction with rotating second magnet.

### Object of the invention

Accordingly, object of the invention is a mixing device according to claim 1. A process for mixing a biological sample like a cell suspension is also disclosed by providing a biological sample in a liquid to a mixing vessel, submerging a mixing rod in the liquid and stir the liquid by a first magnet (1) rotating around a longitudinal axis (2); a mixing rod (4) attached to a mount (6), the mount including a second magnet (3), wherein the mount (6) moves in a substantially orthogonal motion to the longitudinal axis of the mixing rod (4) by the interaction of the rotating first magnet with the second magnet (3).

With the device of the invention, it is possible to suspend particles even in small volumes of fluid without loss of particles or fluid due to spilling or re-agglomeration .

Within the scope of the invention, the terms "mixing" or "suspending" are synonym and are directed to provide a suspension of particles like cells in a fluid.

The device according to the invention is especially useful for suspending particles like a biological sample in a liquid or fluid by the movement of the mixing rod (4) in the biological sample. "Biological sample" can be any type of tissue or cells to be suspended in an appropriate fluid like cell nutrition buffer.

### Brief description of the drawings:

- Fig. 1: shows the device with first magnet (1), first longitudinal axis (2), second magnet (3), mixing rod (4), mount (6, 6') and second axis (5).
- Fig. 2: shows the first magnet (1) mounted on electric motor (8) with counter weight (9) rotating around first longitudinal axis (2')
- Fig. 3: shows second magnet (3) attached to the mount of mixing rod (4); hinged to joints (6, 6'). The joints are fixed by through holes (7), thereby movement of the mount is limited as depicted by dashed line 6". Mixing rod (4) is guided by sleeve (10).
- Fig. 4: side view of the mixing system with electric motor (8) and mixing rod (4)
- Fig. 5: front view of the mixing system with the hinged joints (6, 6') for the mixing rod (4) removed. Mixing rod (4) is guided by holder or sleeve (10)
- Fig. 6: Examples of movement of the mixing rod (4) (thin line) and resulting convection currents of fluids (bold line)

### Detailed description of the invention

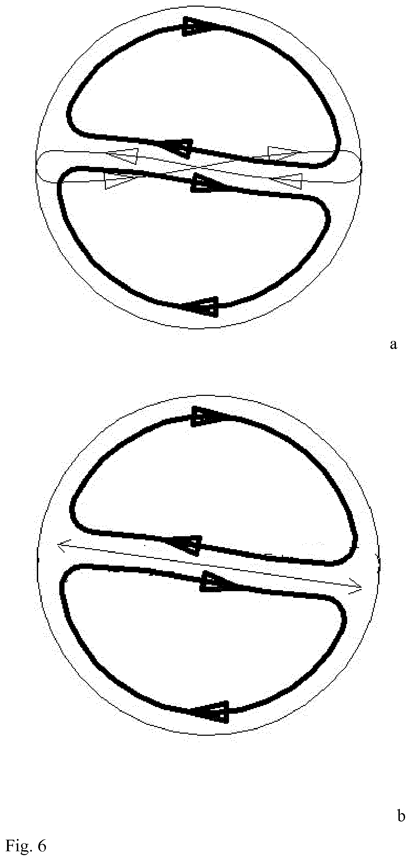

The preferred movement of the mixing rod (4) through the fluid is substantially orthogonal or lateral in view of the longitudinal axis of the mixing rod. Such movement and the resulting currents in the liquid is depicted in Fig. 6 with arrows indicating the direction of movement. By moving the mixing rod (4) (thin lines) through the fluid, convection currents of fluid (bold lines) result with a common zone of turbulence. The currents flow with opposite direction and share at least one common zone of turbulence.

Essential for the mixing device according to the invention is the movement of the mixing rod (4) about the second axis (5). The term "substantially" means that the movement may deviate slightly from the lateral or movement. For example, in Fig. 6a, the drawing shows in thin line a substantial lateral paths of movement of the mixing rod. It is preferred that the movement of the mixing rod (4) is lateral i.e. orthogonal to the longitudinal axis of the mixing rod (4) as shown in Fig. 6b.

In the mixing device according to the invention, in quiescent state of the first magnet the longitudinal axis (2) of the first magnet may by substantially coaxial with a rotational axis of the second magnet (3).

The movement of the mixing rod originates from the rotation of the first magnet (1) around first axis (2). This rotational movement is transferred by magnetic interaction between the first magnet (1) and the second magnet (3) to the mount (6) and finally to the mixing rod (4). Magnetic interaction between the first magnet (1) and the second magnet (3) requires that the distance between the both magnets is sufficiently small. Depending on the orientation of the poles of the first and second magnet, the interaction may generate attracting or opposing forces. In the present invention, first and second magnets are preferable orientated with the same pole facing each other (N-N or S-S). The rotational movement of first magnet (1) and the opposing forces between the magnets "pushes" second magnet (3) into motion.

First magnet (1) and the second magnet (3) are permanent magnets without any special requirements.

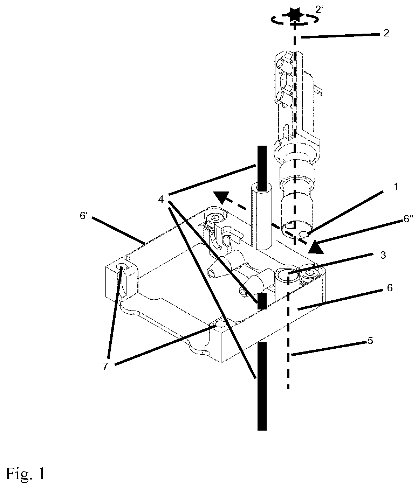

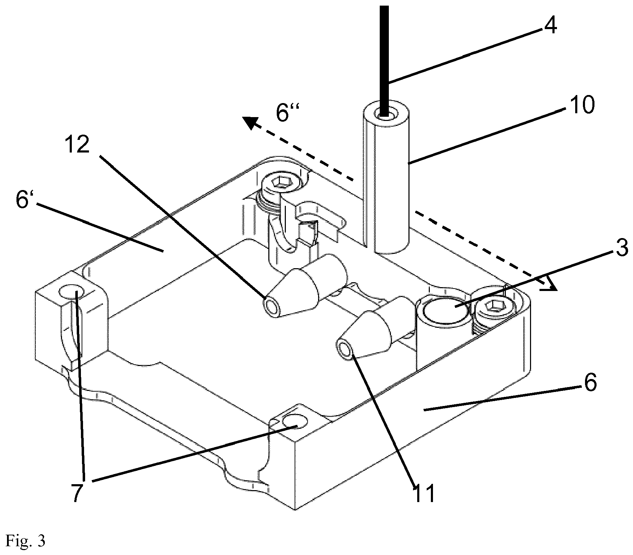

In Fig. 1 and 3, the mixing rod (4) is guided by sleeve or guidance (10) which is attached to mount or hinge (6, 6'). The moving path of the mixing rod (4) is defined or restricted by the mount (6, 6') that allows movement of the second magnet (3) about a second axis (5). According to the invention, the mount is provided as holder with one or two sheet-like hinges (6,6') which can only swing or vibrate about the second axis (5).

In a preferred embodiment of the mixing device, mixing rod (4) is coupled to a mount (6) by a sleeve (10), wherein the sleeve allows movement of the mixing rod along its longitudinal axis. The orthogonal motion of the mixing rod may be accommodated by a plurality of joints disposed on at least two sides of the mount (6). This embodiment is shown in Fig. 3 and results in a substantial lateral movement of the mixing rod (4) along dashed line 6".

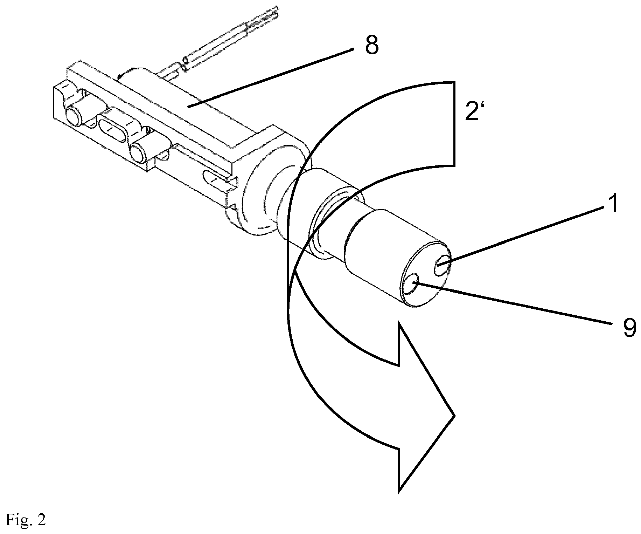

As shown in Fig. 2, rotation of the first magnet (1) is accomplished by an electric motor (8) which rotates around a first axis (2). The first magnet (1) may be fixed in line with the first axis (2) or in a preferred embodiment, asymmetric to the first axis (2) i.e. positioned with a distance of 1 - 5 mm to the first axis (2). In this embodiment, preferable a counterweight (9) to the first magnet (1) is positioned at the same distance to the first axis (2) to prevent imbalance.

The mixing rod (4) is manufactured from a material like stainless steel or the like in contrast to flexible materials like polymers, rubber etc. The mixing rod (4) may be solid or provided as tube or cannula.

If provided as tube or cannula, the mixing rod (4) may be used for filling the mixing vessel with the particles and fluids to be mixed/suspended. In another variant of the invention, the particles (biological sample/cells) and/or the fluid are at least in part inserted or removed from the mixing vessel through a mixing rod (4) provided as tube. Particles and fluids may already be premixed before being provided into the mixing vessel. For this purpose, mixing rod (4) is optionally provided with appropriate connectors like the Luer-system for connection with tubing set.

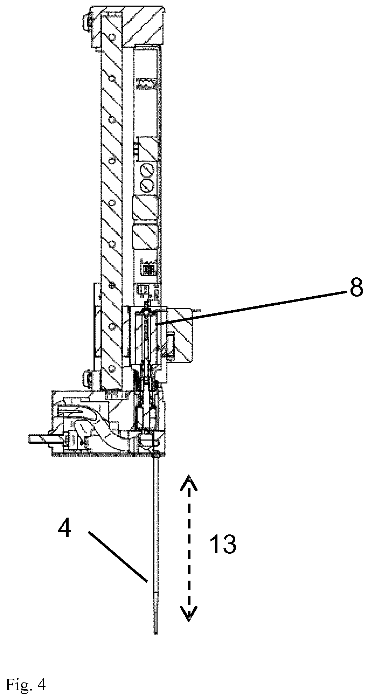

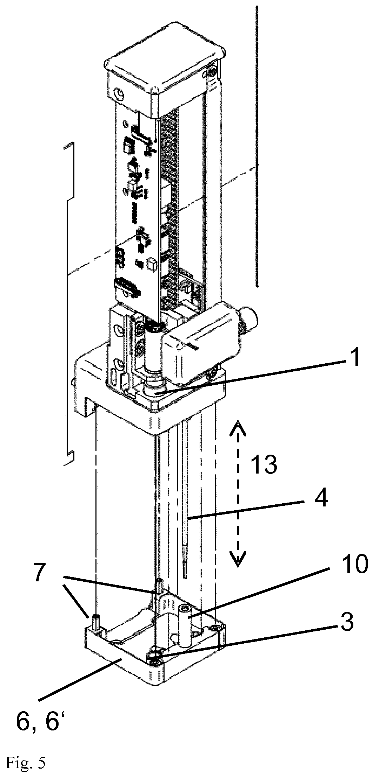

In yet another embodiment of the invention, the mixing device is provided with at least one mixing vessel and the mixing rod can be inserted into and withdrawn from the mixing vessel. Inserting/withdrawing of the mixing rod from the vessel requires either moving the mixing vessel relative to a stationary mixing rod or the mixing rod relative to a stationary mixing vessel. This variant is shown in Fig. 4 and 5, with the position of mixing rod (4) being adjustable relative a not shown mixing vessel in direction of dashed line (13).

In a first variant of this embodiment of the invention, the mixing device is provided with at least one mixing vessel containing a liquid below the mount (6), such that the mixing rod is submerged in the liquid so as to stir the liquid by the orthogonal motion. In this variant, the mixing device further comprises a driver 14 coupled to the motor 8, that stops and reverses the motion of the mixing rod (4), as shown in Fig. 2.

In a second variant of this embodiment of the invention, the mixing device is provided with at least one mixing vessel and the mixing rod (4) is inserted and removed from the mixing vessel by moving the mixing vessel in the direction along the longitudinal axis of the mixing rod.

As mixing vessels, the wells of commercially available microplates are preferred. Of course, the movement of the second magnet (3) and mixing rod (4) about the second axis (5) needs to be adjusted to the space available within the vessel. The mixing rod shall mix the liquid without touching the walls of the vessel throughout its movement.

The method is preferable performed in a mixing vessel wherein the ratio of the outer diameter of the mixing rod (4) and the maximum inner width of the mixing vessel is between 0.1 and 0.3. For example, the mixing rod may have an outer diameter of 1.5 to 0.3 mm and may be utilized in mixing vessels with an maximum inner width of 1 to 10 mm.

The device of the invention can be used for mixing or suspending subsequently a plurality of different samples. It is another object of the invention to avoid contamination of a sample to be suspended by traces or drops of a different sample adhering to the mixing rod.

In further embodiments of the invention, the surface of the mixing rod is cleaned or stripped from adhering mixture by a cleaning pod or a cleaning liquid. For this purpose, the mount (6) may be provided with at least a cleaning pod that removes material from the mixing rod. The cleaning pod may be made from any material suitable for soaking up liquids like cotton or tissue paper.

Better cleaning of the mixing rod can be achieved by using a cleaning liquid and washing the mixing rod. In this embodiment of the invention, the mount (6) is provided with at least one orifice (11, 12) which allows the introduction of a liquid into the sleeve (10). The mixing rod is then guided through a reservoir of the cleaning liquid or through a stream of cleaning liquid, thereby assuring that the mixing rod is only in contact with fresh, uncontaminated cleaning liquid.

Fig. 3 shows this embodiment of the invention, where joint or hinge (6, 6') is provided with sleeve or guidance (10) for the mixing rod (4) which can be flushed with cleaning liquid provided and removed through orifices (11) and (12).

The device according to the invention is especially useful for sample preparation in automated processing or analyzing of biological samples. Usually, such processing requires a homogenous suspension of the sample in a fluid. At best, the homogenous sample is suspended in the fluid shortly before processing.

Accordingly, a process for providing a suspension is also disclosed, comprising: placing particles like a biological sample and a fluid in a mixing vessel; placing the mixing rod of the mixing device as described in the mixing vessel and suspending the particles in the liquid/fluid. The mixing rod may thereby mix the biological sample.

The process can be utilized within high-throughput automates or robots for analyzing a plurality of biological samples. Therefore, multi-well microplates are preferable used as mixing vessel in the process and the mixing rod is inserted in each well /mixing vessel for mixing and withdrawn after suspending/mixing is completed.

The device of the invention can be used for parallel processing of a plurality of samples. For example, suspending of a plurality of biological samples on multi-well microplates may be performed in parallel by 2 to 6 devices of the invention. For this purpose, the mixing device may comprise an additional 1 to 6 mixing rods (4) and mounts (10) which are driven in parallel.

On the other hand, the mixing device may be used for larger volumes by using one mount (6) which includes a plurality of mixing rods (4).

The mixing device of the invention can be used for any automated processing or automated analyzing of biological samples in suspension or solution, especially ELIZA or FACS systems.

## Claims

1. A mixing device comprising:
a first magnet (1) rotating around a longitudinal axis (2);
a mixing rod (4) attached to a mount, the mount including a second magnet (3) having a rotational axis, wherein the longitudinal axis (2) of the first magnet is substantially coaxial with the rotational axis of the second magnet (3), **characterized in that** the mount is provided as holder with one or two sheet-like hinges (6,6') which swing or vibrate about a second axis (5) such that the mount moves in a substantially orthogonal motion (6") to the longitudinal axis of the mixing rod (4) by magnetic interaction of the rotating first magnet with the second magnet (3).

2. The mixing device according to claim 1, **characterized in that** the mixing rod is a tube.

3. The mixing device according to claim 1 or 2, **characterized in that** the mixing rod (4) is coupled to a mount by a sleeve (10), wherein the sleeve allows movement of the mixing rod along its longitudinal axis.

4. The mixing device according to any of the claims 1 to 3, characterized that mount is provided with at least one orifice (11, 12) which allows the introduction of a liquid into the sleeve (10).

5. The mixing device according to any of the claims 1 to 3, characterized that mount is provided with at least a cleaning pod that removes material from the mixing rod.

6. The mixing device according to any of the claims 1 to 5, **characterized in that** at least one mixing vessel containing a liquid is provided below the mount, such that the mixing rod (4) is submerged in the liquid so as to stir the liquid by the orthogonal motion.

7. The mixing device according to any of the claims 1 to 5, **characterized in that** at least one mixing vessel is provided and the mixing rod (4) is inserted and removed from the mixing vessel by moving the mixing vessel in the direction along the longitudinal axis of the mixing rod (4).

8. The mixing device according to any of the claims 1 to 7, wherein the mixing rod (4) does not touch the walls of the vessel throughout its movement.

9. The mixing device according to any of the claims 6 to 8, wherein the outer diameter of the mixing rod (4) is between about 0.1 and 0.3 of a diameter of the vessel.

10. The mixing device according to any of the claims 1 to 9, further comprising an additional 1 to 6 mixing rods (4) and mounts which are driven in parallel.

11. The mixing device according to any of the claims 1 to 10, wherein the mount includes a plurality of mixing rods (4).

12. The mixing device according to any of the claims 1 to 11, further comprising a driver that stops and reverses the motion of the mixing bar.

13. The mixing device according to any of the claims 1 to 12 wherein the mixing rod (4) mixes a biological sample.

## Patentansprüche

1. Mischvorrichtung umfassend:
einen ersten Magneten (1), der sich um eine Längsachse (2) dreht;
einen Mischstab (4), der an einer Halterung befestigt ist, wobei die Halterung einen zweiten Magneten (3) umfasst, der eine Drehachse aufweist, wobei die Längsachse (2) des ersten Magneten im Wesentlichen koaxial zur Drehachse des zweiten Magneten (3) ist,
**dadurch gekennzeichnet, dass** die Halterung als Halter mit ein oder zwei plattenartigen Scharnieren (6,6') vorgesehen ist, die um eine zweite Achse (5) schwingen oder vibrieren, dass die Halterung sich durch magnetische Wechselwirkung des ersten rotierenden Magneten mit dem zweiten Magneten (3) in einer im Wesentlichen orthogonalen Bewegung (6'') zur Längsachse des Mischstabes (4) bewegt.

2. Mischvorrichtung nach Anspruch 1, **dadurch gekennzeichnet, dass** der Mischstab ein Rohr ist.

3. Mischvorrichtung nach Anspruch 1 oder 2, **dadurch gekennzeichnet, dass** der Mischstab (4) mit einer Halterung durch eine Muffe (10) verbunden ist, wobei die Muffe Bewegungen des Mischstabes entlang seiner Längsachse ermöglicht.

4. Mischvorrichtung nach einem der Ansprüche 1 bis 3, **dadurch gekennzeichnet, dass** die Halterung mit mindestens einer Öffnung (11, 12) versehen ist, die die Einführung einer Flüssigkeit in die Muffe (10) ermöglicht.

5. Mischvorrichtung nach einem der Ansprüche 1 bis 3, **dadurch gekennzeichnet, dass** die Halterung mit mindestens einem Reinigungspad versehen ist, das Material vom Mischstab entfernt.

6. Mischvorrichtung nach einem der Ansprüche 1 bis 5, **dadurch gekennzeichnet, dass** mindestens ein Mischbehälter mit einer Flüssigkeit unter der Halterung vorgesehen ist, sodass der Mischstab (4) in die Flüssigkeit getaucht ist, um die Flüssigkeit durch die orthogonale Bewegung zu rühren.

7. Mischvorrichtung nach einem der Ansprüche 1 bis 5, **dadurch gekennzeichnet, dass** mindestens ein Mischbehälter vorgesehen ist und der Mischstab (4) in den Mischbehälter durch Bewegen des Mischbehälters entlang der Längsachse des Mischstabes (4) eingeführt und entfernt wird.

8. Die Mischvorrichtung nach einem der Ansprüche 1 bis 7, wobei der Mischstab (4) die Wände des Behälters während seiner gesamten Bewegung nicht berührt.

9. Die Mischvorrichtung nach einem der Ansprüche 6 bis 8, wobei der Außendurchmesser des Mischstabes (4) ungefähr 0,1 und 0,3 des Durchmessers des Behälters ist.

10. Die Mischvorrichtung nach einem der Ansprüche 1 bis 9, ferner umfassend zusätzliche 1 bis 6 Mischstäbe (4) und Halterungen, die parallel betrieben werden.

11. Die Mischvorrichtung nach einem der Ansprüche 1 bis 10, wobei die Halterung mehrere Mischstäbe (4) umfasst.

12. Die Mischvorrichtung nach einem der Ansprüche 1 bis 11, ferner umfassend einen Antrieb, der die Bewegung des Mischstabes stoppt und reversiert.

13. Die Mischvorrichtung nach einem der Ansprüche 1 bis 12, wobei der Mischstab (4) eine biologische Probe mischt.

## Revendications

1. Dispositif de mélange comprenant :
un premier aimant (1) rotatif autour d'un axe longitudinal (2) ;
une tige de mélange (4) fixée à un support, le support comprenant un deuxième aimant (3) présentant un axe de rotation,
dans lequel l'axe longitudinal (2) du premier aimant est substantiellement coaxial avec l'axe de rotation du deuxième aimant (3),
**caractérisé en ce que** le support se présente comme un dispositif de maintien avec une ou deux charnières du genre feuille (6, 6'), lesquelles oscillent ou vibrent autour d'un deuxième axe (5), de telle façon que le support se déplace dans un mouvement substantiellement orthogonal (6") par rapport à l'axe longitudinal de la tige de mélange (4) par interaction magnétique du premier aimant rotatif avec le deuxième aimant (3).

2. Dispositif de mélange selon la revendication 1, **caractérisé en ce que** la tige de mélange est un tube.

3. Dispositif de mélange selon la revendication 1 ou 2, **caractérisé en ce que** la tige de mélange (4) est accouplée à un support par un manchon (10), le manchon permettant le mouvement de la tige de mélange le long de son axe longitudinal.

4. Dispositif de mélange selon l'une quelconque des revendications 1 à 3, **caractérisé en ce que** le support est pourvu d'au moins un orifice (11, 12) permettant d'introduire un liquide dans le manchon (10).

5. Dispositif de mélange selon l'une quelconque des revendications 1 à 3, **caractérisé en ce que** le support est pourvu d'au moins une cosse de nettoyage éliminant du matériau sur ka tige de mélange.

6. Dispositif de mélange selon l'une quelconque des revendications 1 à 5, **caractérisé en ce que** l'au moins un récipient de mélange contenant un liquide est disposé sous le support, de telle façon que la tige de mélange (4) est plongée dans le liquide de manière à remuer le liquide par le mouvement orthogonal.

7. Dispositif de mélange selon l'une quelconque des revendications 1 à 5, **caractérisé en ce qu'**il est prévu au moins un récipient de mélange et la tige de mélange (4) est insérée et retirée du récipient de mélange en déplaçant le récipient de mélange dans la direction le long de l'axe longitudinal de la tige de mélange (4).

8. Dispositif de mélange selon l'une quelconque des revendications 1 à 7, dans lequel la tige de mélange (4) ne touche pas les parois du récipient durant son mouvement.

9. Dispositif de mélange selon l'une quelconque des revendications 6 à 8, dans lequel le diamètre extérieur de la tige de mélange (4) est compris entre environ 0,1 et 0,3 d'un diamètre du récipient.

10. Dispositif de mélange selon l'une quelconque des revendications 1 à 9, comprenant en outre 1 à 6 tiges de mélange (4) et supports supplémentaires entraînés en parallèle.

11. Dispositif de mélange selon l'une quelconque des revendications 1 à 10, dans lequel le support comprend une pluralité de tiges de mélange (4).

12. Dispositif de mélange selon l'une quelconque des revendications 1 à 11, comprenant en outre un dispositif de commande destiné à arrêter et inverser le mouvement de la barre e mélange.

13. Dispositif de mélange selon l'une quelconque des revendications 1 à 12, dans lequel la tige de mélange (4) mélange un échantillon biologique.
